# EUROPEAN PATENT APPLICATION

(11) **EP 0 658 624 A2**
(43) Date of publication of application: **21.06.1995**
(21) Application number: 94119226.2
(22) Date of filing: 06.12.1994
(51) Int. Cl.: C12N 15/12, C07K 14/47, A61K 38/17, C12N 15/62, A61K 47/28, A61K 31/57, C12N 1/21

(54) **Recombinant genes expressing pp14**

(30) Priority: 08.12.1993 US 162957
(71) Applicant: INTERMUNE LIFE SCIENCES, INC., Etobicoke, Ontario M9W 5S6 (CA)
(72) Inventor: Bolton, Anthony E, Quebec, JO1 1LO (CA); Dalton, Caroline Frances, Sheffield, S10 5BA (GB)
(74) Representative: Patentanwälte Ruff, Beier, Schöndorf und Mütschele

(57) **Abstract**

A recombinant expression vector containing a DNA sequence encoding PP14 wherein the vector is capable of expressing PP14 in a transformed microorganism, and therapeutically active fragments of said PP14. Also disclosed is a transport mechanism whereby recombinant or isolated and purified natural PP14 binds with progesterone or other small molecules and delivers said molecules to target cells.

SEQUENCE LISTING

(1) GENERAL INFORMATION:

(i) APPLICANT: Intermune Life Sciences, Inc.

(ii) TITLE OF INVENTION: Recombinant Genes Expressing PP14

(iii) NUMBER OF SEQUENCES: 1

(iv) CORRESPONDENCE ADDRESS:
(A) ADDRESSEE: Ruff, Beier und Partner
(B) STREET: Willy-Brandt-Strasse 28
(C) CITY: D-70173 Stuttgart
(D) STATE:
(E) COUNTRY: Federal Republic Germany
(F) ZIP:

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: EP 94119226.2
(B) FILING DATE: 6-Dec-1994
(C) CLASSIFICATION:

(viii) ATTORNEY/AGENT INFORMATION:
(A) NAME: Muetschele, T.
(B) REGISTRATION NUMBER:
(C) REFERENCE/DOCKET NUMBER: A 29 358 EP

(ix) TELECOMMUNICATION INFORMATION:
(A) TELEPHONE: 49-711-29 95 81
(B) TELEFAX: 49-711-29 95 86

(2) INFORMATION FOR SEQ ID NO:1:

(i) SEQUENCE CHARACTERISTICS:
(A) LENGTH: 819 base pairs
(B) TYPE: nucleic acid
(C) STRANDEDNESS: double stranded
(D) TOPOLOGY: linear

(x) PUBLICATION INFORMATION:

(A) AUTHORS: Julkunen, Mervi
Seppälä, Markku
Jänne, Olli A.

(B) TITLE: Complete Amino Acid Sequence of Human
Placental Protein 14: a Progesterone-Regulated
Uterine Protein Homologous to ß-Lactoglobulins

(C) JOURNAL: Proc. Natl. Acad. Sci. USA
(D) VOLUME: 85
(F) PAGES: 8845-8849
(G) DATE: DEC-1988
(K) RELEVANT RESIDUES IN SEQ ID NO:1: FROM 1 to 819

(xi) SEQUENCE DESCRIPTION: SEQ ID NO:1:

CATCCCTCTG GCTCCAGAGC TCAGAGCCAC CCACAGCCGC AGCC 44

ATG CTG TGC CTC CTG CTC ACC CTG GGC GTG GCC 77
Met Leu Cys Leu Leu Leu Thr Leu Gly Val Ala
-18 -10

CTG GTC TGT GGT GTC CCG GCC ATG GAC ATC CCC 110
Leu Val Cys Gly Val Pro Ala Met Asp Ile Pro
1

CAG ACC AAG CAG GAC CTG GAG CTC CCA AAG TTG 143
Gln Thr Lys Gln Asp Leu Glu Leu Pro Lys Leu
10

GCA GGG ACC TGG CAC TCC ATG GCC ATG GCG ACC 176
Ala Gly Thr Trp His Ser Met Ala Met Ala Thr
20

AAC AAC ATC TCC CTC ATG GCG ACA CTG AAG GCC 209
Asn Asn Ile Ser Leu Met Ala Thr Leu Lys Ala
30

CCT CTG AGG GTC CAC ATC ACC TCA CTG TTG CCC 242
Pro Leu Arg Val His Ile Thr Ser Leu Leu Pro
40

ACC CCC GAG GAC AAC CTG GAG ATC GTT CTG CAC 275
Thr Pro Glu Asp Asn Leu Glu Ile Val Leu His
50

AGA TGG GAG AAC AAC AGC TGT GTT GAG AAG AAG 308
Arg Trp Glu Asn Asn Ser Cys Val Glu Lys Lys
60 70

GTC CTT GGA GAG AAG ACT GGG AAT CCA AAG AAG 341
Val Leu Gly Glu Lys Thr Gly Asn Pro Lys Lys
80

TTC AAG ATC AAC TAT ACG GTG GCG AAC GAG GCC 374
Phe Lys Ile Asn Tyr Thr Val Ala Asn Glu Ala
90

ACG CTG CTC GAT ACT GAC TAC GAC AAT TTC CTG 407
Thr Leu Leu Asp Thr Asp Tyr Asp Asn Phe Leu

TTT CTC TGC CTA CAG GAC ACC ACC ACC CCC ATC 440
Phe Leu Cys Leu Gln Asp Thr Thr Thr Pro Ile
110

CAG AGC ATG ATG TGC CAG TAC CTG GCC AGA GTC 473
Gln Ser Met Met Cys Gln Tyr Leu Ala Arg Val
120

CTG GTG GAG GAC GAT GAG ATC ATG CAG GGA TTC 506
Leu Val Glu Asp Asp Glu Ile Met Gln Gly Phe
130

ATC AGG GCT TTC AGG CCC CTG CCC AGG CAC CTA 539
Ile Arg Ala Phe Arg Pro Leu Pro Arg His Leu
140

TGG TAC TTG CTG GAC TTG AAA CAG ATG GAA GAG 572
Trp Tyr Leu Leu Asp Leu Lys Gln Met Glu Glu
150

CCG TGC CGT TTC TAG CTCACCTCCG CCTCCAGGAA 607
Pro Cys Arg Phe AM
160 162

GACCAGACTC CCACCCTTCC ACACCTCCAG AGCAGTGGGA CTTCCTCCTG 657

CCCTTTCAAA GAATAACCAC AGCTCAGAAG ACGATGACGT GGTCATCTGT 707

GTCGCCATCC CCTTCCTGCT GCACACCTGC ACCATTGCCA TGGGGAGGCT 757

GCTCCCTGGG GGCAGAGTCT CTGGCAGAGG TTATTAATAA ACCCTTGGAG 807

CATGAAAAAA AA 819

## Description

### BACKGROUND OF THE INVENTION

### (1) Field of the Invention

The present invention relates to recombinant genes capable of expressing placental protein 14 (PP14) and methods of producing the same.

### (2) Description of the Related Art

Human endometrium synthesizes several proteins under the influence of progesterone. Of these proteins, placental protein 14 (PP14) is of particular interest, as it is also present in circulation, and the PP14 concentrations in serum have been used as indicators of endometrial function. Although PP14 was initially isolated from human term placenta and hence named placental protein 14, subsequent studies indicate that its main site of synthesis during pregnancy is the decidua. By immunological criteria, PP14, progestogen-dependent endometrial protein, and pregnancy-associated endometrial α₂-globulin are indistinguishable and appear to represent different names for a single protein. See, M. Julkunen et al., "Complete amino acid sequence of human placental protein 14 ....", Proc. Nat'l Acad. Sci., Vol. 85, pp 8845-8849 (December 1988).

Of the range of proteins which have been described as associated with the pregnant state, PP14 has been found to exhibit an immunosuppressive activity in a variety of *in vitro* tests. U.S. Patent 5,039,521, incorporated herein by reference discloses that the mode of action of this peptide has indicated that it inhibits IL-1 production by peripheral white blood cells (containing both T-lymphocytes and monocytes) after stimulation. The concentration at which this protein is active appears to be the levels at which it is found normally in pregnancy.

The time course of this inhibition of IL-1 production closely relates to the immunosuppressive activity of the molecule, indicating that its primary effect is on monocytes rather than other immune system cells.

Unlike many other pregnancy-associated and placental proteins, which are present at maximal concentration in maternal serum at about 4-6 weeks before parturition, and whose material serum levels consequently tend to correlate with placental weight, e.g., pregnancy-associated α₂-glycoprotein, PP5 and hPL, circulating levels of PP14 fall during the whole of the third trimester and therefore are not related directly to placenta weight. A.E. Bolton, et al., "The radioimmunoassay of human placental protein 14 (PP14)", Clinica Chemica Acta, p. 283-291 (1983), discloses that the mean concentration of this protein in serum of 22 normal pregnant women showed a decline during the third trimester from 120 µg/1 at 27 weeks gestation to 65 µg/1 at term. Serum samples from 16 patients with intra-uterine growth retardation tended to contain lower concentrations of placental protein 14, these results reaching significance at weeks 36-38 of gestation. Of seven patients with pre-eclampsia from whom two or more blood samples were taken, four showed increases in concentration of this protein as pregnancy proceeded, compared with the normal pattern of decreasing values.

As discussed, by A. Bolton and A. Drizen in U.S. Patent 5,039,521, interleukin 1 (IL-1) is a peptide cytokine secreted by a range or variety of cell types including accessory cells of the immune system, the antigen presenting cells, and has a variety of functions including an involvement in the activation of immune system T-cells. Cells secreting IL-1 include monocytes present in the circulating blood, macrophages found in interstitial fluid, and dendritic cells. It now appears established that IL-1 is a central mediator of inflammatory reactions and is important in the pathogenesis of chronic inflammatory reactions and diseases, of which rheumatoid arthritis (RA) is one example.

It is becoming widely accepted that IL-1 (both IL-1 alpha and IL-1 beta) are important mediators of inflammatory responses. IL-1 appears to directly cause cartilage breakdown in knee joints, and may be central in the pathogenesis of rheumatoid arthritis. An inhibitor may, therefore, be of importance in treatment of this disease. It is of interest that PP14 is a natural product present at elevated levels in peripheral circulation early in pregnancy, peaking around week 9-10, and there are reports in the literature that there is a marked improvement in some sufferers with rheumatoid arthritis in the first trimester of pregnancy. Similar reports of an improvement of patients with chronic asthma in the first trimester of pregnancy can also be found in the published literature. Chronic asthma is an inflammatory disease, and although IL-1 has not yet been implicated in its etiology, this remains a possibility.

As PP14 appears early in pregnancy, it may be associated with the process of implantation and in maintaining the early conceptus which may be particularly prone to immune rejection by the maternal immune system. It is possible that PP14 could be of utility in the treatment of early miscarriage, which may relate to immune phenomena.

Pregnancy is a normal state in which at least one aspect of the immune response -- reaction to foreign antigens -- is suppressed in regard to paternal antigens expressed by the fetus. It is rational, therefore, to seek natural inhibitory regulators of the immune response in the tissue or bloodstream of pregnant women. The expression of a variety of proteins is induced to high levels during pregnancy. One of these, PP14, is a major secretory protein of decidual tissue, where it comprises about 10% of the total soluble protein.

One difficulty that has been encountered with work related to PP14 has been the inability to produce the protein in sufficient large quantities. A variety of natural sources, including mammalian placenta, mammalian blood, amniotic fluid, seminal plasma, cells in tissue culture, decidual cells, decidual organs, endometrial cells, and endometrial organs have been used as sources to date.

The publication to M. Julkunen et a discussed above discloses the isolation of PP14 from human decidual cDNA clones and deduced its amino acid sequence. A reliable technique for preparing high yields of PP14 using a microorganism as the source of supply, however, has remained unavailable until the present invention.

### Brief Description of the Drawings

Figure 1 depicts a side view of a three-dimensional computer generated structural model of PP14.

Figure 2 depicts an end view of a three-dimensional computer generated structural model of PP14.

Figure 3 depicts a side view of a three-dimensional computer generated space filling representation of PP14.

Figure 4 depicts an end view of a three-dimensional computer generated space filling representation of PP14.

Figure 5 depicts the SDS-polyacrylamide gel electrophoresis pattern of recombinant PP14.

### Summary of the Invention

The present invention relates to a microorganism transformed with a recombinant expression vector containing a DNA sequence encoding PP14 which enables PP14 to be prepared in commercially available quantities.

In one aspect, the invention involves a recombinant expression vector containing a DNA sequence encoding PP14, wherein the vector is capable of expressing PP14 in a transformed microorganism, and preferably, wherein the vector is pGEX-KG and the microorganism is Escherichia coli.

In another aspect the invention involves a method of producing a microorganism transformed with a vector encoding PP14, which comprises: preparing a ligation vector containing a DNA sequence encoding PP14; preparing an expression vector containing a replaceable insert gene for another protein; cutting the DNA sequence encoding PP14 out of the ligation vector and discarding the ligation vector; cutting the replaceable insert gene out of the expression vector and discarding the replaceable insert gene; and ligating the DNA sequence encoding PP14 and the expression vector to form a plasmid capable of expressing PP14.

Preferred embodiments involve use of an expression vector which is pGEX-KG; wherein the recovered plasmid is pGEX-KG with the cDNA gene sequence for PP14; wherein the plasmid is recovered using a fusion protein; and wherein the fusion protein is glutathione S-transferase.

Another embodiment involves the recombinant protein itself produced by the microorganism.

Another embodiment involves derivatives, muteins, fragments and subunits of the recombinant PP14 produced by the organism.

### Description of the Preferred Embodiment

In its broadest aspects the invention involves the preparation and recovery of a recombinant PP14 protein. The overall process involves isolating RNA from tissue such as endometrial tissue, and then converting the RNA to DNA. The DNA is then duplicated, such as by PCR (polymerase chain reaction) and isolated on a gel by electrophoresis. The recovered gene is then ligated into a ligation vector which forms a cDNA (PP14) gene vector. An expression vector is separately prepared using pGEX-KG to prepare an expression system that can be used by the microorganism. Inserted into the expression vector is another protein, which is removed when the expression vector is cut. The ligation vector is then cut and the PP14 gene in the ligation vector replaces the protein insert in the expression vector. The expression vector or plasmid now contains the pGEX-KG vector with the PP14 cDNA gene. The other fractions containing the ligation vector and the insert protein are removed and discarded.

The plasmid vector is then infected into E. coli, with or without a drug resistant fragment, such as ampicillin resistance. The microorganism is also infected with another plasmid to code for the amino acid arginine. The arginine encoding plasmid is essential for the microorganism to prepare PP14 since PP14 is rich in arginine and E. Coil are arginine deficient.

Once the recombinant PP14 has been synthesized by the microorganism it may be recovered in conventional ways to purify proteins. One particularly preferred purification mechanism involves forming a fusion protein with glutathione S-transferase (GST), and the PP14 protein, passing the fusion protein through a chromatographic column which binds to the glutathione S-transferase, cleaving the fusion protein bond to the GST with thrombin and then eluting the recombinant PP14.

While the process of the invention has been described herein using microorganisms and particularly E. coli, other cell systems may be used to prepare the recombinant PP14 described herein, e.g. yeast cells, insect cells and mammalian cells.

The recombinant PP14 may be used for treating an immune system disorder in a human by administering to the human the PP14 or a substance selected from the group consisting of derivatives of PP14, fragments of PP14, muteins of PP14 and subunits of PP14, in an amount effective to alleviate the disorder. The disorders which may be treated by this method include allergic conditions, auto-immune conditions, and inflammatory conditions.

The substance may be administered to the patient by any appropriate route, including intravenous injection, intramuscular injection, oral administration, topical administration, rectal administration, and inhalation. The substance may be administered in admixture with pharmaceutically acceptable carriers.

Specific immune system disorders which may be treated according to this method include arthritis, rheumatoid arthritis, asthma, graft-versus-host disease, organ rejection osteoarthritis, systemic lupus erythematosus, atopic allergy, multiple sclerosis, allergic dermatitis, inflammatory bowel disease, psoriasis, sarcoidosis, and other inflammatory disorders.

In another embodiment, the immune system disorder to be treated according to the present invention may be a lymphoproliferative disorder, such as malignant non-Hodgkins lymphoma, Hodgkin's disease, or malignant histiocystotis. The PP14 may also be used to treat infertility disorders and prevent miscarriage.

Therapeutically useful derivatives of PP14 may be prepared by augmenting the primary amino acid sequence of the protein PP14 with at least one additional molecule selected from the group consisting of glucose moieties, lipids, phosphate groups, acetyl groups, hydroxyl groups, saccharides, methyl groups, propyl groups, amino acids, and polymeric molecules. Augmentation may be accomplished through post-translational processing systems of the producing host, or it may be carried out *in vitro*. Both techniques are well-known in the art.

Referring to SEQ ID NO:1 for the sequence description of PP14, it should be noted that the peptide includes three potential glycosylation sites at amino acid residues 28-30, 63-65, and 85-87. Glycosylation is a process of forming a protein derivative, wherein a portion of the protein's amino acid sequence is augmented by a sugar moiety. It will therefore be understood that therapeutically useful derivatives of PP14 may be prepared by addition of one or more sugar residues to the protein, or alternatively by removal of some or all of the sugar residues from the sites of glycosylation on the PP14 molecule.

Other therapeutically useful derivatives of PP14 may be formed by modifying at least one amino acid residue of PP14 by oxidation, reduction, or other derivatization processes known in the art.

Muteins of PP14 which do not destroy the activity of the protein may be used as the active treating substance of the instant invention. Muteins are prepared by modification of the primary structure of the protein itself, by deletion, addition, or alteration of the amino acids incorporated into the sequence during translation. For example, at least one cysteine residue of PP14 may be replaced with a conservative amino acid, in order to eliminate sites of undesirable intramolecular disulfide bond formation. Crosslinking is undesirable if it changes the conformation of PP14 so as to render the protein essentially inactive for purposes of treating an immune system disorder. Also, it may be desirable to replace a methionine which is not essential to bioactivity with a conservative amino acid alteration which is defined as one which does not significantly adversely affect biological activity and involves substitutions of the amino acid. The conservative amino acid that may be substituted for cysteine and methionine include at least: serine, alanine, glycine, vane, threonine, leucine, isoleucine, and tyrosine. More preferably they include serine and alanine. Most preferably, cysteine may be replaced with serine and methionine replaced with alanine.

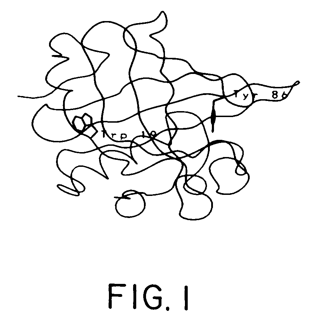

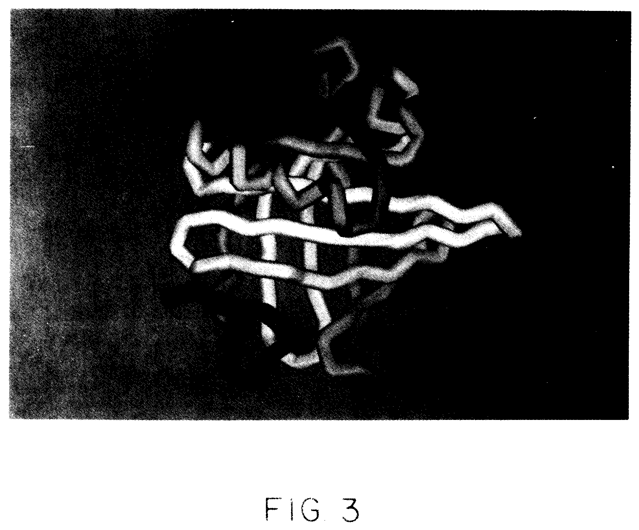

Placental protein 14 is believed to exist in nature as a dimer of two identical, non-covalently linked protein subunits. Accordingly, since each subunit is believed to have the amino acid sequence shown in SEQ ID NO:1 and the structure shown in Figures 1 through 4, a subunit of PP14 could be used as the therapeutic active substance for treating human immune system disorders according to the instant invention. The invention also encompasses use of subunits of PP14 that are covalently linked, either naturally or by artificial techniques known in the art.

In addition, it is contemplated that fragments of PP14 would be useful for treating human immune system disorders, provided that such fragments retained their therapeutic activity. From molecular modeling based on the known amino acid sequence of PP14 shown in SEQ ID NO:1 and the structures of related proteins, the fragments which are believed to be biologically active have been identified.

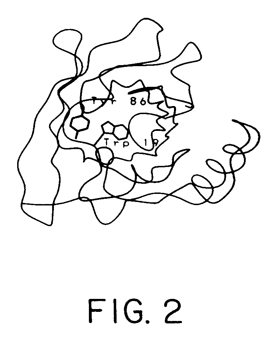

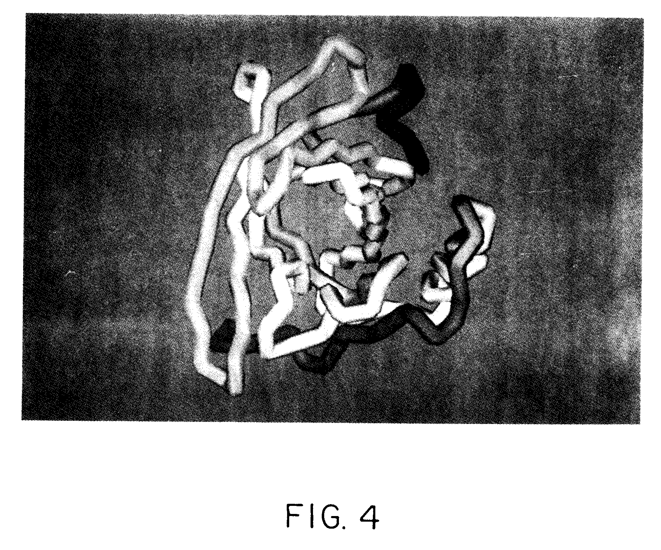

PP14 is believed to be a member of a family of proteins called lipocalins, which are characterized by the presence of a binding pocket for small lipophilic molecules. Other lipocalins include β-lactoglobulin, retinol-binding protein and major urinary protein. Referring to Figures 1 and 2, the three-dimensional computer-generated structures of PP14 depicts a binding pocket with the tryptophan residue at position 19 in SEQ ID NO:1 at the bottom. This binding pocket is also apparent in the center of the space-filling representation shown in Figure 4. In other members of the lipocalin family, the tryptophan at position 19 is involved with binding a small molecule and signaling to a receptor. Thus, the loop of the PP14 molecule defined by residues 17-21, including tryptophan 19, is believed to be involved in recognition and receptor binding.

The other loops on this side of the molecule share the greatest homology with the other members of the lipocalin family, which usually means that the mechanism of action is similar, although subtle differences within each sequence prevent cross-reaction with the receptors of each molecule. The greatest homology is in the loop defined by residues 97-103. It is believed that this loop is a possible receptor. Another loop on this side is defined by residues 130-140, an area of partial homology with other lipocalins, and is believed to be the part of the molecule that ensures the specificity of the binding of PP14 to its receptors.

The fragment defined by amino acid residues 83-90 is another site of biological activity. The tyrosine residue at position 86 in SEQ ID NO:1 is at the top of the binding pocket, in common with other lipocalins. This residue can swing to open or shut the pocket like a trap-door. Another loop in this region, defined by amino acid residues 112 to 118, does not have homology with other lipocalins but is believed to be another binding site.

The mechanism of PP14's interaction with other small molecules is not completely known. It is believed, however, that PP14's immunological activity may be keyed to a transport mechanism whereby it binds with a ligand in the binding pocket described above, interacts with receptors on the target cells, and delivers the ligand to the target cells where the ligand, rather than the PP14 itself, exerts its immunoregulatory effect on the target cells. Known target cells include monocyte/macrophages and natural killer (NK) cells.

It is further believed that progesterone, a known immunosuppressive agent at high concentrations, binds to the PP14 binding pocket. It should be recognized that other small molecules having similar structure may be involved in this transport mechanism as well. By identifying the region of the PP14 molecule that interacts with the receptors on the target cells, a delivery system specific for the target cells could be developed. One possible use of a targeting technology for monocyte/macrophages and NK cells could be their stimulation in cancer immunotherapy.

The above-described forms of PP14 are used in an effective therapeutic amount, which will vary depending on the particular immune system disorder being treated, the method of administration, the form of PP14 utilized, and other factors understood to those having ordinary skill in the art. For example, treatment of arthritis may require direct injection of PP14 into the joints in order to achieve a suitable therapeutic dose, whereas a topically administered preparation for the treatment of psoriasis may require a different dosage able to arrive at therapeutically effective dosages of PP14 for treatment of the various immune system disorders contemplated by the invention, based on the range of concentrations of PP14 which has been discovered to provide suitable *in vitro* activity, namely from about 0.1 to about 10 micromole/liter.

Another aspect of the invention relates to a method of improving the efficacy of assisted reproductive techniques, such as artificial insemination (AI), *in vitro* fertilization (IVF), and gamete intra-fallopian transfer (GIFT), by adding an effective amount of PP14 to any sample (embryo, fertilized or unfertilized egg, mixture of eggs and sperm, or sperm alone) that is to be introduced into the uterus in order to induce a pregnancy.

A description of assisted reproductive techniques is found in Tan et al, Infertility: Your Questions Answered (McGraw-Hill Book Co., 1991), the disclosure of which is incorporated herein by reference.

*In vitro* fertilization is a technique whereby one or more mature eggs (oocytes) are removed from the woman's ovary, fertilized outside of the body by sperm either from the husband or from a donor, then transferred back into the uterus. The fertilized eggs which are transferred into the uterus are also called "pre-embryos". A similar process is embryo transfer (ET), in which embryos are formed outside of the uterus then transferred into the uterus. An "embryo" is generally defined as the state of a fertilized egg two or more weeks after fertilization.

Gamete intra-fallopian transfer (GIFT) is a treatment for women who have patent (not blocked) fallopian tubes. The first few steps of GIFT are similar to those in IVF, namely, stimulation of the ovary with drugs, monitoring follicular growth, administration of human chorionic gonadotrophin (hCG) to induce maturation of the eggs, and collection of the eggs. After collection of the eggs, the maturity of the eggs is observed, then sperm and eggs re separately drawn into a catheter and deposited into the patient's fallopian tubes. The resulting pre-embryo then moves down the fallopian tubes and into the uterus where implantation occurs. Thus, the primary distinction between GIFT and IVF is that in GIFT, fertilization occurs within a fallopian tube, whereas in IVF fertilization occurs in a laboratory.

Direct intra-peritoneal insemination (DIPI) involves stimulating the patients' ovaries with drugs, observing the development of the follicles, administering hCG to induce egg maturation, then injecting a washed sample of semen through the top of the vagina into the Pouch of Douglas. Peritoneal oocyte and sperm transfer (POST) is a similar treatment in which eggs and washed sperm are both transferred into the Pouch of Douglas proximate to the opening of the fallopian tubes.

Zygote intra-fallopian tube transfer (ZIFT), also known as pronuclear stage tubal transfer (PROST), is an *in vitro* fertilization process wherein fertilized eggs are transferred into fallopian tubes by laparoscopy when the pronuclei form (at about 18 hours following fertilization). Tubal embryo transfer (TET), also known as tubal embryo stage transfer (TEST), is a similar process; however, transfer of the fertilized eggs into the fallopian tubes is not carried out until the pre-embryo has divided into two ore more cells.

In direct oocyte transfer (DOT), eggs are collected according to IVF, then the eggs are incubated for a few hours prior to the addition of sperm. Eggs are transferred into the uterus using IVF once sperm are seen binding to the eggs.

Trans-uterine fallopian transfer (TUFT) involves the transfer of a pre-embryo into the fallopian tube using a catheterizing instrument.

Artificial insemination (AI) is a procedure in which sperm is introduced into the patient's genital tract by artificial means rather than by intercourse. Insemination may be either into the cervix or into the uterine cavity itself (intra-uterine insemination, IUI).

Unwashed semen is generally not employed in IUI because the protective cervical mucus barrier is bypassed in this process, and unwashed sperm would create a risk of infection or allergic reactions. Also, prostaglandins present in the semen may cause contractions of the uterus which may be painful and cause ejection of semen from the uterus.

Washing of the sperm is carried out by repeated extractions in appropriate solvents, followed by centrifugation to recover the sperm. Alternatively, the semen may be mixed with a cleaning solvent, and the healthy sperm permitted to swim to the surface of the solvent where they are collected for use in the AI or IVF process.

PP14 is found in relatively high concentrations in seminal plasma. However, as discussed above, sperm used for artificial insemination (AI) and other assisted reproductive techniques are frequently washed prior to use in order to remove prostaglandins present in the seminal plasma. This conventional washing step results in the removal of PP14 from the seminal plasma. The invention therefore contemplates the addition of recombinant PP14 to sperm which has been washed, to restore PP14 to levels which exhibit *in vitro* activity.

For example, before sperm is introduced into a patient by AI, recombinant PP14 may be added to the sperm sample, preferably by including the recombinant PP14 in the medium used to dilute the washed sperm prior to insemination. Similarly, recombinant PP14 may be added to washed sperm before use in other assisted reproductive techniques, in order to replace PP14 which is lost during the conventional washing stage.

Fertile women have been found to exhibit a concentration of PP14 in flushings from the uterine lumen of about 79 ng/ml on day 7 after ovulation. See T.C.Li et al. "Concentration of Endometrial Protein PP14 in Uterine Flushings Throughout the Menstrual Cycle in Normal Fertile Women". British Journal of Obstetrics and Gynaecology, May 1993 Vol. 100, p. 460-464. Moreover, in any assisted reproductive technique, whether or not washed sperm are being employed, the addition of extra medium into the uterus along with the gametes inevitably dilutes the normal PP14 concentration present in the uterus. The invention thus contemplates adding an effective amount of recombinant PP14 to the gamete medium of the like, so as to provide PP14 concentration having *in vitro* activity when subjected to the testing procedures described below. In general, adding recombinant PP14 to the sample in an amount ranging from about 0.1 µg/ml to about 5 mg/ml, and preferably from about 5 µg/ml to about 5 mg/ml, is contemplated by the invention.

It will be understood that the amount of recombinant PP14 added to the sample to be introduced into the patient to induce pregnancy will vary depending, for example, on the level of PP14 already present in the patient, the particular assisted reproductive technique employed, whether or not washed sperm are employed, the volume of the sample to be introduced into the patient, and other factors understood to those or ordinary skill in the art.

As discussed, the PP14 may be added to any sample that is to be introduced into the reproductive tract of the patient. For example, PP14 may be added to an embryo, fertilized or unfertilized egg, mixture of eggs and sperm, or sperm alone, and may be added prior to, concurrently with, or following introduction of the sample into the patient. It is believed that the potential efficacy of PP14 in preventing miscarriage in women who have experienced recurrent miscarriage is related to the immuno-suppressive activity of natural PP14.

The following examples are given to illustrate the invention, but are not to be limiting thereof. All percentages given throughout the specification are based upon weight unless otherwise indicated. All protein molecular weights are based on mean average molecular weights unless otherwise indicated.

### Collection of Tissue Sample

A 0.2g endometrium biopsy sample was taken from a healthy, fertile women. The sample was removed using a Sherman's curette which removes a piece of the fundus of the endometrium. The biopsy was placed into a small sterile container and snap frozen immediately in liquid nitrogen. It was then transferred to a -70°C freezer and stored until required.

### Isolation of mRNA

mRNA was extracted from the biopsy sample using the method of Chomcznski and Sacchi Analytical Biochemistry 162, 156-159 (1987), which procedure is incorporated herein by reference. 100mg of biopsy sample was placed in a 1.5ml tube and kept on ice. 1ml of denaturing solution (4M guanidinium thiocyanate, 25mM sodium citrate, pH 7, 0.5% sarkosyl, 0.1 M β-mercaptoethanol) was added and the tissue gently homogenized until the tissue had broken down and a uniform suspension had been obtained. The tissue suspension was then split into 2 aliquots and each portion placed in a 1.5ml tube. 10ml of 2M sodium acetate buffer pH 4.0 was added to each tube. After mixing, 1ml of phenol saturated with water was added, the tubes were again mixed by inversion of 200µl of chloroform:isoamyl alcohol mixture (49:1) was added. The tubes were shaken vigorously for 10 seconds, then chilled on ice for 20 minutes. After centrifuging at 10,000g for 20 minutes at 4°, the upper aqueous layers containing RNA were transferred to fresh tubes. An equal volume (650µl) of ice-cold isopropanol was added to each tube and the samples were cooled at -20°C for 1 hour to precipitate the RNA. The RNA was pelleted by centrifuging the tubes at 10,000g for 20 minutes at 4°C. The supernatant was then removed and discarded. The pellets were resuspended in 300µl of denaturing solution, then 300µl of isopropanol was added and after mixing the tubes were again called at 20°C for 1 hour. After centrifuging at 10,000g for 10 minutes at 4°C, the supernatant was again removed and discarded. 100µl of 75% ethanol was added without resuspending the pellet and the samples were frozen at -70°C until required for the reverse transcriptase reaction.

### Design of primers

Two synthetic oligonucleotides were designed from the published sequence of PP14 depicted in SEQ ID NO:1. See M. Julkunen et a. ECoR I and Hind III sites were designed into the primers to enable the insertion of the PCR product into the multiple cloning site of the pGEX-KG expression vector. They were checked for the formation of hairpin loops, and were synthesized on an Applied Biosystems 392 DNA/RNA synthesizer. The primer sequences were:
5'-CGCGGAATTCGCATGCTGTGCCTCCTGCTC-3' specific for the 5' end incorporating an EcoR I site (underlined) and
5'-GCGCGAAGCTTCTAGAAACGGCACGGCTC-3' specific for the 3' end incorporating a Hind III site (underlined).

The absorbance of a diluted solution of the primers was measured at 260nm in a spectrophotometer. The extinction coefficient of the primers was calculated using the total molarity of the oligonucleotide and from these the molarity of the primer solution was calculated.

### Reverse Transcriptase

One of the purified mRNA samples was thawed on ice, the 75% ethanol removed and the mRNA dried under vacuum in a evaporation centrifuge.

The pellet was resuspended in 8µl of a 0.1 % DEPC (dimethyl pyrocarbonate) treated water. This was estimated to have approximately 3mg mRNA in it: To this was added 5µl bulk strand reaction mixture (containing reverse transcriptase, BSA, dATP, dCTP, dGTP and dTTP in aqueous buffer), 1µl of 3' primer containing 20pmol of DNA and 1µl of 200mM DTT. (Reagents provided by Pharmacia as the "First-Strand cDNA Synthesis Kit".) The reactants was incubated for 1 hour at 37°C and then used in PCR (polymerase chain reaction) reaction.

### PCR Reaction

The PCR reaction was performed with the following reaction mixture:
1µl cDNA (1/1, 1/10, 1/100, 1/1000 dilutions)
2µl dNTPs diluted to 100mM
2µl 3' primer (diluted to 10µM)
2µL 5' primer (diluted to 10µM)
10µl PCR buffer
0.75µl T*aq* polymerase
All the reagents except the T*aq* polymerase were assembled in a 500µl tube and heated to 100°C for 5-10 minutes. After cooling on ice for a few minutes the T*aq* polymerase was added and after gentle mixing the reaction mixture was overlayed carefully with 100µl of light mineral oil. The tubes were placed in a thermal cycler (Coy Tempcycler, Flogen) and the PCR reaction cycle performed 30 times. Each cycle consisted of a denaturing step at 94°C for 45 seconds, a primer annealing step at 50°C for 2 minutes and an extension step at 72°C for 90 seconds. The tubes were then kept at 4°C.

### Agarose gel electrophoresis

The PCR reaction components were separated by electrophoresis on an agarose gel. A 1% gel was cast by melting 0.75g of agarose in 75ml of TAE (40mM Tris-Acetate, 1mM EDTA) buffer in a conical flask in a microwave. The hot gel mixture was cooled slightly by holding a flask under a running cold tap, then the gel was poured into a casting frame sealed at either end with masking tape. The samples were prepared by adding sample buffer (0.25% bromophenol blue, 0.25% xylene cyanol FE, 15% Ficoll, Type 400 in water) to them (e.g., 25ml of sample buffer added to 100µl of sample). After the gel had set the samples were loaded. Cut ECoR1 and Hind III were run as markers. The gel was run for 1 hour at 100V with TAE as running buffer. The gel was removed from the mold and placed in a container with 100ml of water and 100µl of a 10mg/ml solution of ethidium bromide (EtBr) and gently agitated for 20 minutes. The EtBr was then washed off and the gel observed under UV light and photographed. The band containing the amplified DNA was carefully excised from the gel and placed in a pre-weighted 1.5ml tube to be genecleaned.

### Genecleaning of gel slices

The DNA was purified from the gel slice using the GeneClean kit from Bio 101. This involves binding the DNA to glass beads "glassmilk" and then washing the beads with an ethanol-based wash solution "newwash", then eluting the DNA from the beads with water. The tube was weighed and the weight of the gel calculated. 3ml of Nal was added for every g of gel and the tubes were incubated at 50°C until the gel had dissolved (approx. 15 minutes). The tubes were then cooled on ice for 10 minutes. 5µl of "glassmilk" was added and the tubes were left on ice for 15 minutes. During this time they were vortexed every 5 minutes. The tubes were centrifuged for 2 minutes and the supernatants carefully removed. The pellet was resuspended in 500µl of "newwash" and recentrifuged. The supernatants was again removed. This step was repeated 2 times. Finally the DNA was eluted from the glass beads by resuspending the pellet in 10µl of Milli Q water and incubating at 50^{o}C for 5 minutes. After centrifuging for 10 seconds the supernatant was removed and retained and the resuspension, incubation and centrifugation was repeated. The second supernatant was pooled with the first.

### Blunt ended ligation of PCR product

The genecleaned PCR product was ligated in pUC 18 using a Sureclone kit from Pharmacia as follows:
The quantity of DNA obtained after the genecleaning procedure was estimated by running an agarose gel of the sample with markers of known quality of DNA and comparing the intensity of the bands obtained. 100ng of PCR product was prepared for ligation using a blunting/kinasing reaction. The reaction mixture contained 16µl of PCR product containing 100ng of DNA, 1µl of Klenow fragment, 2ml of buffer and 1µl of Polynucleotide Kinase. The reaction mixture was incubated at 37°C for 30 minutes, then 20ml of phenol/chloroform was added, the mixture was vortexed, then centrifuged for 1 minute. The upper layer was purified on an S-200 Microspin column.

### Ligation into pUC

The purified blunt-ended DNA was ligated into a vector for 2 hours at 16°C. The ligation mixture contained:
7µl DNA
10µl ligation buffer
1µl pUC 18 vector containing 50ng DNA
1µl DTT
1µl T4 Ligase
A control ligation mixture containing all the reagents except the DNA which was replaced by water was treated in an identical manner. After the ligation the reactions were placed on ice until they were transformed into competent E. coli cells.

### Preparation of competent E. coli

5ml of Luria Broth LB medium (10g tryptone, 5g yeast extract and 10g NaCl in 1 liter H₂O) was inoculated from an original stock of JM 109 E. Coli cells (available from Stratagene). The cells were grown overnight at 37°C with vigorous shaking in a loosely capped culture tube. 50ml of LB containing 10mM MgSO4 and 0.2% glucose (medium A) in a 100ml flask was inoculated with 0.5ml of the overnight culture. The flask was shaken vigorously at 37°C with good aerating for 2-1/2 hours. The cells were placed on ice for 10 minutes then pelleted by centrifuging at 1500g for 10 minutes at 4°C. The cells ware resuspended gently in 0.5ml of medium A and 2.5ml of a storage medium of LB containing 36% glycerol, 12% PEG 8000 and 12mM MgSO4 and 100µl aliquots were placed in 1.5ml eppendorf tubes. The tubes were stored at -70°C until used. The viability of the cells was tested before using them for transformations by streaking a plate of LB with the cells and observing the growth at 37°C overnight.

### Transformation of E. Coli

Competent JM 109 cells were transformed by removing them from the - 70°C freezer and placing the tubes immediately on ice. The cells were allowed to just thaw and a portion of the ligation mixture was added (5µl). Aliquots of cells containing the control ligation mixture without the insert, the vector only and no DNA were also prepared as controls. The tubes were gently mixed and left on ice for 15-30 minutes. The cells were then heat-shocked by placing them in a 42°C water bath for exactly 2 minutes. After replacing the tubes on ice for 2 minutes 1 ml of pre-warmed LB was added to each tube, the tubes were gently mixed by inversion and the cells were incubated at 37°C for 45-60 minutes. The tubes were centrifuged at room temperature (24°C) for 15-40 seconds and some of the supernatant was removed to leave 200µl of medium in the tube. At this point, for transformations of pUC, 18 40µl of 200mM isopropylthio-B-D-galactoside (IPTG) and 40µl of 20 mg/ml 5-bromo-4-chloro-3-idolyl-B-D-galactoside (X-Gal) was added to each tube of cells. Transformations of pGEX did not have any further reagents added to the cells. The cells were gently resuspended in the remaining medium and plated onto Lₐₘₚ plates. The plates were incubated overnight at 37°C.

### Identification of transformants

The plates were inspected the next morning for colonies. When ligating into pUC the colonies containing the PCR product are white due to the inactivation of the IacZ' gene. Colonies containing religated vector without the insert still have the active IacZ' gene the product of which, B-galactosidase converts X-Gal blue when induced by IPTG. This provides a blue/white selection for recombinants since colonies without the insert will be blue. When cloning into pGEX the incidence of false positives is much higher since there is no way of screening between positive and negative religated vector. Up to 36 colonies from the plate with the ligation mixture containing the insert were picked off the plate using an inoculation loop and grown up overnight in 5ml of Lₐₘₚ broth at 37°C within loosely capped culture tubes with vigorous shaking.

### Minipreparation of plasmid DNA

1.5ml of each overnight culture was placed in a 1.5ml tube and the cells pelleted by centrifuging for 1 minute. The cells were resuspended by vigorous vortexing in 100µl of ice-cold miniprep I solution (25mM Tris-HCl pH8 containing 50mM glucose and 10mM EDTA). After leaving the tubes at room temperature for 5 minutes with the lids open 200µl of fresh miniprep II solution (0.2M NaOH, 1% SDS in H₂O) was added, the tubes mixed by inversion and placed on ice for 5 minutes. Following this 150µl of ice-cold miniprep III (60ml 5M potassium acetate, 11.5ml acetic acid, 28.5ml water) solution was added and the tubes again left on ice for 5 minutes. After briefly vortexing the tubes they were centrifuged for 5 minutes and the supernatants transferred to fresh tubes. An equal volume (450µl) of phenol was added, the tubes were vortexed thoroughly and centrifuged for 5 minutes. The supernatants were removed and discarded and 1 ml of ethanol at - 20°C was added. The tubes were left on ice for 10 minutes, then centrifuged for 7 minutes. The supernatants were removed and discarded and the pellet was washed with 100µl of 70% ethanol. The pellet was dried using a vacuo centrifuge and then resuspended in 20µl of water.

### Cutting out the insert from the transformant with restriction enzymes

Plasmid DNA from the minipreps was digested with restriction enzymes to cut out the PCR product if it was present. The reaction mixture consisted of:
12 Units EcoRI
12 Units Hind III
2µl incubation buffer (100mM Tris-HC1 pH 7.5, 50mM NaCl, 6mM MgC12, 6mM B-mercaptoethanol)
1µl of RNase (10mg/ml, previously boiled for 15 minutes)
10µl of miniprep DNA
5ml of Milli Q water
The reaction was carried out at 37°C for 1 hour and 10µl of the reaction mixture was then run on an agarose gel as detailed above.

Positive clones were identified from the gel by the presence of a band in addition to the plasmid band the same size as the insert.

### Preparation of insert from positive colonies

Further cultures of the positive clones were grown up and the DNA miniprepped on exactly the same way. The DNA was digested with restriction enzymes using the following reaction mixture:
40µl DNA
12 Units ECoR1
12 Units Hind III
8µl buffer
26µl Milli Q water
The DNA was then run on a gel and the band containing the insert was excised and genecleaned as described above.

### Preparation of pGEX vector

An overnight culture of E. Coli (JM 109) containing the vector pGEX obtained from the stock preparation in the laboratory was grown up in 2 x 5ml loosely capped cultures tubes overnight at 37°C with vigorous shaking. Minipreparations of plasmid DNA were made as described above.

### Digestion of nGEX and with restriction enzymes

The DNA was digested with restriction enzymes using the following reaction mixture:
40µl DNA
12 Units ECoR1
12 Units Hind III
8µl buffer
26µl water
The reaction was carried out for 1.5 hours at 37°C and then separated on an agarose gel as detailed. The band containing the plasmid was excised from the gel as detailed. The band containing the plasmid was excised from the gel and genecleaned as above.

### Ligation

The insert was religated into pGEX without insert using the following ligation mixture:
1µl pGEX containing 6.4ng of DNA
1µl insert containing 20ng of DNA
1µl ligation buffer (300mM Tris-HCl, pH 7.8, 100mM MgCl₂, 100mM
DTT, 10mM ATP)
0.5µl T4 DNA ligase
6.5µl Milli Q water
The reaction was carried out at 4°C for 16 hours. The ligation mixtures were transformed into competent cells as described previously and the plates prepared and incubated as before.

### Transformation of plasmid argU into positive recombinant clones.

The next morning up to 48 colonies were picked off the plate containing the ligation mixture with the insert in and miniprepped as described. The positive clones were identified by running a gel and looking for the insert.

Positive clones containing PP14 in pGEX were used to prepare competent cells as previously described. The cells were checked for viability. The cells were transformed as described above by the addition of 1µl of the argU plasmid. This time the cells were plated onto twin selection plates containing ampicillin and kanamycin.

Because the vector does not contain an insert and has not been digested there is no possibility of false positives due to re-ligated vector therefore all colonies on the plates will contain both plasmids.

### Small scale expression of recombinant protein

5ml overnight cultures of clones were grown overnight and 50µl inoculated into 5ml of fresh medium. The cultures were grown up for 2 hours at 37°C and 1ml of uninduced culture removed. Protein expression was induced by the addition of 1mM IPTG. 1ml of culture medium was removed every hour for 4 hours after induction. Control cultures without the insert were also induced. The samples were spun for 1 minute to pellet the cells and the cells were resuspended in 100µl SDS running buffer.

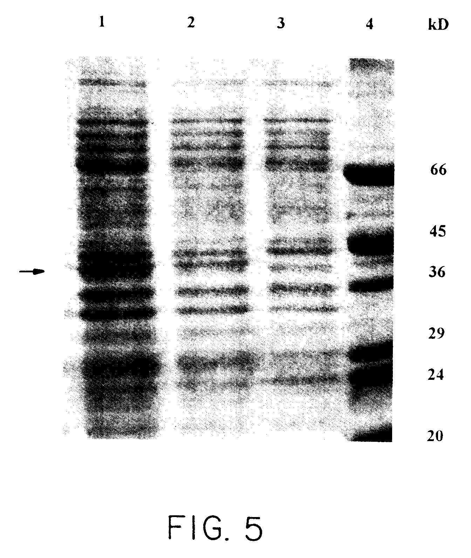

The samples were then heated to 100°C for 3 minutes then spun for 1 minute. 10µl of the sample was loaded onto the 12% SDS gel and electrophoresis performed. Figure 5 depicts the SDS-polyacrylamide gel electrophoresis pattern of the recombinant PP14, with the induced fusion protein GST-PP14 marked with an arrow. Lane 1 contains pGEX-KG containing PP14 insert induced with 1mM IPTG for 2 hours. Lane 2 contains pGEX-KG containing PP14 insert induced with 1 mM IPTG for 1 hour. Lane 3 contains pGEX-KG containing PP14 insert before induction. Lane 4 contains molecular weight markers.

The invention being thus described, it will be obvious that the same may be varied in many ways. Such variations are not to be regarded as a departure from the spirit and scope of the invention, and all such modifications are intended to be included within the scope of the following claims.

## Claims

1. A recombinant expression vector containing a DNA sequence encoding PP14, wherein the vector is capable of expressing PP14 in a transformed microorganism.

2. The expression vector of claim 1, wherein the vector is pGEX-KG.

3. The expression vector of claim 1 or 2, wherein the transformed microorganism is Escherichia coli.

4. A microorganism transformed with the vector of claim 1, wherein said microorganism is capable of expressing PP14.

5. The microorganism of claim 4 which is Escherichia coli.

6. A DNA isolate consisting essentially of a DNA sequence encoding PP14.

7. A composition consisting of DNA molecules which encode PP14.

8. The recombinant protein produced by the expression vector of claim 1.

9. Fragments of the recombinant protein of claim 8.

10. The fragments of claim 9, wherein said fragments have the nucleotide and amino acid sequence of residues 17 through 21 of SEQ ID NO:1.

11. The fragments of claim 9, wherein said fragments have the nucleotide and amino acid sequence of residues 83 through 90 of SEQ ID NO:1.

12. The fragments of claim 9, wherein said fragments have the nucleotide and amino acid sequence of residues 97 through 103 of SEQ ID NO:1.

13. The fragments of claim 9, wherein said fragments have the nucleotide and amino acid sequence of residues 112 through 118 of SEQ ID NO:1.

14. The fragments of claim 9, wherein said fragments have the nucleotide and amino acid sequence of residues 130 through 140 of SEQ ID NO:1.

15. The recombinant protein produced by the microorganism of claim 4.

16. Fragments of the recombinant protein of claim 15.

17. The fragments of claim 16, wherein said fragments have the nucleotide and amino acid sequence of residues 17 through 21 of SEQ ID NO:1.

18. The fragments of claim 16, wherein said fragments have the nucleotide and amino acid sequence of residues 83 through 90 of SEQ ID NO:1.

19. The fragments of claim 16, wherein said fragments have the nucleotide and amino acid sequence of residues 97 through 103 of SEQ ID No:1.

20. The fragments of claim 16, wherein said fragments have the nucleotide and amino acid sequence of residues 112 through 118 of SEQ ID NO:1.

21. The fragments of claim 16, wherein said fragments have the nucleotide and amino acid sequence of residues 130 through 140 of SEQ ID NO:1.

22. A method for producing a microorganism transformed with a vector encoding PP14, which comprises:
preparing a ligation vector containing a DNA sequence encoding PP14;
preparing an expression vector containing a replaceable insert gene for another protein;
contacting the ligation vector with the expression vector to form a plasmid capable of expressing PP14 and another vector containing the replaceable insert gene;
and recovering the plasmid capable of expressing PP14.

23. The method of claim 22, wherein the expression vector is pGEX-KG.

24. The method of claim 22 or 23, wherein the recovered plasmid is pGEX-KG with the cDNA for PP14.

25. The method of one of claims 22 to 24, wherein the plasmid is recovered using a fusion protein.

26. The method of claim 25, wherein the fusion protein is glutathione S-transferase.

27. A composition comprising purified PP14 in combination with a small molecule.

28. The composition of claim 27, wherein the small molecule is progesterone.

29. The composition of claim 27 or 28, wherein said purified PP14 is recombinant PP14.

30. The composition of claim 27 or 28, wherein said PP14 is isolated and purified natural PP14.

31. A method for treating an immune system disorder, which comprises:
administering to a patient an effective amount of a complex comprising PP14 coupled to a small molecule.

32. The method of claim 31, wherein the small molecule is progesterone.

33. The method of claim 31 or 32, wherein the PP14 is isolated and purified natural PP14.

34. The method of claim 31 or 32, wherein the PP14 is recombinant PP14.

35. The use of a complex comprising PP14 and a small molecule, which comprises: using said complex in an effective amount for treating an immune system disorder.

36. The use of the complex of claim 35, wherein the small molecule is progesterone.

37. The use of the complex of claim 35 or 36, wherein the PP14 is isolated and purified natural PP14.

38. The use of the complex of claim 35 or 36, wherein the PP14 is recombinant PP14.

39. The method as defined in one of claims 31 to 34, wherein said immune system disorder is selected from the group consisting of allergic conditions, autoimmune conditions, and inflammatory conditions.

40. The use defined by one of claims 35 to 38, wherein said immune system disorder is selected from the group consisting of allergic conditions, autoimmune conditions, and inflammatory conditions.

41. A method of preventing miscarriage in a female by administering to said female a therapeutically effective amount of a complex comprising a small molecule of PP14, derivatives of PP14, muteins of PP14, fragments of PP14, and subunits of PP14, so as to prevent miscarriage in the female.

42. The method of claim 41, wherein said derivatives of PP14 comprise PP14 augmented by at least one additional molecule selected from the group consisting of glucose moieties, lipids, phosphate groups, acetyl groups, hydroxyl groups, saccharides, methyl groups, propyl groups, amino acids, and polymeric molecules.

43. The method of claim 41 or 42, wherein the small molecule is progesterone.

44. A method of improving the efficacy of an assisted reproductive technique, which comprises adding a therapeutically effective amount of a complex comprising PP14 and a small molecule to a sample that is to be introduced into the uterus of a female in order to induce a pregnancy.

45. The method of claim 44, wherein the assisted reproductive technique is artificial insemination.

46. The method of claim 44, wherein the assisted reproductive technique is in vitro fertilization.

47. The method of claim 44, wherein the assisted reproductive technique is gamete intra-fallopian transfer.

48. The method of one of claims 44 to 47, wherein the sample comprises a fertilized or unfertilized egg.

49. The method of one of claims 44, to 48, wherein the sample comprises a mixture of sperm and at least one egg.

50. The method of one of claims 44 to 49, wherein the sample comprises sperm.

51. The method of one of claims 44 to 50, wherein the PP14 is added to sample before the sample is introduced into an uterus.

52. The method of claim 44, wherein the assisted reproductive procedure is artificial insemination, the sample comprises sperm, and the PP14 complex is added to a medium used to dilute the sperm prior to insemination.

53. The method of one of claims 44 to 52, wherein the PP14 present in the complex to be administered is in an amount of from about 0.1 µg/ml to about 5 mg/ml.

54. The method of one of claims 44 to 53, wherein the female is a human.

55. The method of one of claims 44 to 54, wherein the small molecule is progesterone.
